# EUROPEAN PATENT SPECIFICATION

(11) **EP 2 582 838 B1**
(45) Date of publication and mention of the grant of the patent: **16.07.2014**
(21) Application number: 11723326.2
(22) Date of filing: 10.05.2011
(51) Int. Cl.: C12Q 1/68

(54) **SOLUBLE QUENCHER TO REDUCE BACKGROUND IN QPCR ASSAYS**
LÖSLICHER QUENCHER ZUR HINTERGRUNDREDUKTION BEI QPCR-TESTS
DÉSACTIVATEUR SOLUBLE POUR RÉDUIRE LE BRUIT DE FOND DANS LES DOSAGES DE PCR QUANTITATIVE

(30) Priority: 21.06.2010 EP 10166625
(43) Date of publication of application: 24.04.2013
(73) Proprietor: Siemens Healthcare Diagnostics Inc., Deerfield, IL 60015-0778 (US)
(72) Inventor: SCHWERS, Stephan, 50827 Köln (DE)
(74) Representative: Maier, Daniel Oliver
(86) International application number: PCT/EP2011/057506
(87) International publication number: WO 2011/160887

(56) References cited:
- EP-A1- 1 739 190

## Description

The invention is in the field of analytical technology and relates to an improved procedure for determining presence of a nucleic acid in a sample. Specifically the invention provides methods useful when conducting fluorescence detection methods. In particular, it is useful for conducting (RT-)quantitative PCR (qPCR) reactions for detection of DNA and RNA involving fluorescent probes.

Diagnostic assays for the detection of infectious agents (e.g. viruses, bacteria) are complicated by the fact, that they need to test for a multitude of different analytes to be potentially present in the sample.

Current qPCR instruments can separate at most five different fluorophors, enabling the independent detection of five different analytes in a qPCR reaction. Anyway, it is often only required to detect the presence/absence of an infectious agent of interest, but not to identify it's concrete genotype or species. To this end it is possible to add several specific primer/probesets to the reaction mix, which are all labeled with the same fluorophor: A positive signal in this detection channel would then indicate the presence of (at least) one of those infections agents (without further distinction, which agent it is).

This approach however has a technical limitation: Addition of several probes labeled with one and the same fluorophore increasingly adds to the background of this detection channel. As a result, the fluorescent background signal might be too high for the detection system. In fact, none of the commercially available qPCR instruments is equipped with grey filters or other hardware, which would allow to actually decrease the overall sensitivity in a detection channel.

The document US 6180349 describes a quantitative PCR Method, wherein dual-labeled fluorophore probes are carrying a fluorophore label at one end of the oligonucleotide and an appropriate quencher molecule at the other end. Efficient quenching is only possible, if fluorophore and quencher are brought in close proximity to each other. Even if a quencher is used, the quenching may not be 100%, thus increasing unspecific background noise.

Strategies to reduce the fluoresence background of a dual labeled probe are aiming to minimize the distance between fluorophore and quencher by e.g.:
- Minimizing the overall length of the oligonucleotide
- Introducing an "internal" quencher, which is located inside the oligo sequence (i.e. not at the terminus)
- Provoking secondary structures of the oligonucleotide, that bring FL and quencher in close proximity (e.g. introducing complementary sequences adjacent to FL and quencher, as exemplified in molecular beacons).

All those approaches have disadvantages:
Minimizing the overall oligonucleotide length is only possible to a certain extend, since it affects the specificity and Tm of the probe. Utilizing probes with "Minor Groove Binders" (MGB) or "Locked Nucleic Acids" (LNA) might compensate for the loss of specificity and Tm, but is very costly.

Internal quenchers can influence the probe specificity (through steric hindrance during hybridization) and increase the cost of probe synthesis.

Also Molecular Beacons are more expensive that conventional probes (due to licensing fees and increased overall length of those probes).

The document EP1739190 describes a method of detecting a target nudeic acid wherein methylene blue dye is added to the sample as a soluble light emission modifier. However, methylene blue itself is a fluorophor.

It is an object of the present invention to overcome the above mentioned problems.

### Summary of the invention

The present invention relates to a method for determining the presence of a test nucleic acid in a sample according to claim 1, comprising the steps of
a) amplifying the test nucleic acid by an amplification reaction,
b) labeling the amplified nucleic acid by providing a fluorescent dye label with an excitation wavelength and an emission wave length,
c) adding a soluble quenching dye, said soluble quenching dye having an absorption of at least 40% of its maximal absorbance at the excitation wavelength and/or at the emission wave length of the fluorescent dye label,
d) detecting the labeled nucleic acid dye by detecting emitted light at the emission wavelength of the fluorescent dye label,
wherein the concentration of said soluble quenching dye is 1 % (w/v) or less.

A test nucleic acid is a nucleic acid with a specific sequence to be tested for. The method of the invention is applicable to both to a qualitative and a quantitative determination of the presence of a test nucleic acid in a sample.

A sample can be any sample containing the test nucleic acid, e.g. body fluid sample, blood sample, tissue sample, smear sample, etc.

An amplification reaction may be any suitable amplification reaction, e.g. PCR, SDA, bDNA and others.

A fluorescent dye label is a label for fluorescently labeling an amplified test nucleic acid in a sample.

A soluble quenching dye is any dye suitable for absorbing electromagnetic radiation at the excitation wavelength and/or at the emission wave length of a given fluorescent dye, selected from the group consisting of Bromphenol Blue, Trypan Blue, Methyl Blue, and Remazol Blue.

The excitation wavelength of the fluorescent dye is the local absorption maximum at which fluorescence can be optimally induced or excited.

The emission wave length of the fluorescent dye is the local emission maximum at which fluorescence can be optimally detected.

Under optimal conditions the wave length of maximal absorbance (i.e. the local peak of the absorption spectrum) of the soluble quenching dye coincides with the excitation wavelength and/or at the emission wave length of the fluorescent dye label. However, the invention only requires said soluble quenching dye having an absorption of at least 40% of its maximal absorbance at the excitation wavelength and/or at the emission wave length of the fluorescent dye label in order for the invention to work. Preferably the soluble quenching dye has an absorption of at least 50%, 60%, 70%, 80%, 90% or more at the excitation wavelength and/or at the emission wave length of the fluorescent dye label.

The concentration of said soluble quenching dye is 1 %(w/v) or less. It can be for example 0.1 %(w/v), 0.05 %(w/v), 0.01 %(w/v), 0.005 %(w/v), 0.001 %(w/v), 0.0005 %(w/v), 0.0001 %(w/v), or less, depending on fluorophore and quenching dye used. Essentially, the soluble quenching dye will reduce the fluorescence signal of the fluorescent dye label, thus reducing both background and specific signal. Finding the optimal concentration of soluble quenching dye is a matter of performing a simple titration: Too much quenching dye will result in a quenching of any specific signal, too little will result in no significant reduction of background noise. Essentially, the soluble quenching dye acts like a grey filter specifically for the fluorescent dye label.

According to an aspect of the invention the concentration of said soluble quenching dye is 50 % or less of the concentration of said fluorescent dye label. The concentration of said soluble quenching dye can be 1/2, 1/5, 1/10, 1/20, 1/50, 1/100, 1/200, 1/500, 1/1000, or anywhere between ½ to 1/10.000 or less of the concentration of said fluorescent dye label.

According to an aspect of the invention the amplification reaction is a PCR reaction.

According to an aspect of the invention the fluorescent dye label is an intercalating dye, e.g. ethidium bromide, SYBR Green, etc.

According to an alternative aspect of the invention the fluorescent dye label is bound to a nucleic acid probe capable of specifically binding the test nucleic acid.

The method of the invention can be used both for single labeled probes (fluorophore only) or dual labeled probes (fluorophore and quencher).

According to an aspect of the invention the presence of at least one further test nucleic is determined, wherein said at least one further test nucleic acid is labeled with at least one further fluorescent dye label and wherein said quenching dye has an absorption of at 50% of its maximal absorbance or less (preferably 40%, 30%, 20% or 10% or less) at the excitation wavelength and/or at the emission wave length of said at least one further fluorescent dye label. According to this aspect, a so called multiplex assay is performed, wherein different test nucleic acids are amplified and labeled with different fluorescent dye labels. The quenching dye is conveniently chosen such that the fluorescence of a first fluorescent dye label is dampened (i.e. said soluble quenching dye having an absorption of at least 40% or more at the excitation wavelength and/or at the emission wave length of the fluorescent dye label) while the fluorescence of a second fluorescent dye label is more or less unaffected (said quenching dye has an absorption of at most 40% or less at the excitation wavelength and/or at the emission wave length of said at least one further fluorescent dye label).

According to an aspect of the invention the fluorescent dye label has an excitation wavelength and/or an emission wavelength between 340 and 710 nm, preferably between450 nm and 650 nm. This is the range in which the excitation wavelengths and/or an emission wavelengths of ROX label and other fluoro-phores commonly used in PCR assays are found.

### Description of the invention

The invention is now described in conjunction with examples and figures which show:
- FIG 1: shows the absorption spectrum of a preferred soluble quenching dye, Bromphenol blue (BPB) which can be used in the method of the invention.
- FIG 2: shows absorption spectra of further soluble quenching dyes which can be used in the method of the invention.
- FIG 3: shows fluorescence spectra of soluble quenching dyes which can be used in the method disclosed herein.
- FIG 4 to 8: show experimental data obtained either without use of a soluble quenching dye or with use of a soluble quenching dye according to the method of the invention.

The distinguishing feature of the invention is to decrease the background fluorescence not of an individual probe, but of all probes carrying the same fluorescent label. This is achieved by adding a soluble quenching dye to the qPCR reaction mix. This dye then acts as a "soluble shield" and allows to reduce the fluorescent background brought in by a large number of identically labeled probes. Depending on the nature (i.e. absorption spectrum) of the quenching dye, it is possible to selectively reduce the background of a single detection channel, while maintaining the signal strenght of the other detection channels: This provides more flexibility, in case not all channels exhibit the same high background.

The inventive step lies in tackling the high background problem not on the level of individual probes (as described under 2.), but to reduce fluorescent signal of all those probes at once by addition of a reagent, that is inexpensive and normally used for staining.

Furthermore the quenching dye should
- be soluble in the solvent used for the amplification reaction (e.g. water soluble),
- shield fluorescent light preferably in a single detection channel (while leaving the other channels unaffected) in this case a relatively narrow absorption spectrum is preferable,
- not inhibit the (RT-)qPCR reaction,
- not emit light (i.e. should not be fluorescent) in the emission channel of the fluorescent dye to be shielded or dampened.

The advantage of this approach is a much reduced cost as compared to the current solutions mentioned above. Furthermore, it is possible to adjust the dampening effect of the dye by adjusting its concentration in the reaction mix.

Apart from this invention, other, more obvious possibilities exist to reduce either high assay background or the overall sensitivity of fluorescence detection. However, those solutions would involve the hardware side, e.g. implementation of a light reducing filter or reducing the signal amplification factor of the detection unit, which is expensive and may limit other uses of the detection system. Furthermore, available qPCR instruments are not equipped with such solutions, since they are usually trimmed for maximum sensitivity. Therefore the present invention helps to overcome existing hardware limitations by modification of the assay reaction mix.

The below Table 1 shows some commonly used fluorescent dye labels and excitation and emission wavelengths:

**Table 1**

| Label | Ex | Em |
|---|---|---|
| Alexa350 | 343 | 441 |
| FAM | 495 | 520 |
| HEX | 535 | 556 |
| CAL Fluor Orange 560 | 538 | 559 |
| ROX | 586 | 610 |
| CAL Fluor Red 610 | 590 | 610 |
| Quasar 670 | 647 | 667 |
| Quasar 705 | 690 | 705 |

| | | |
|---|---|---|
| CAL Fluor and Quasar are registered trademarks of Biosearch Technologies, Inc., Novato, CA. | | |

### Polymerase Chain Reaction (PCR)

The Polymerase Chain Reaction (PCR), is a means to amplify the amount of DNA or mRNA fragments, e.g. of a specific gene, in a (patient) sample. If mRNA fragments shall be amplified, they first have to be transcribed to cDNA in a reverse transcription (RT) step. In this case, the reaction is called RT-PCR.

The PCR takes place in small reaction tubes in a thermal cycler. The reaction mix consists of
- the original DNA template which contains the region (target) that should be amplified
- two primers which tag the beginning of the region that should be amplified on the sense respectively anti-sense strand of the DNA
- the (Taq) polymerase which synthesizes new DNA strands
- deoxynucleoside triphosphates (dNTPs) which are the components of the DNA strands to be synthesized
- a buffer solution
- divalent magnesium or manganese cations and monovalent potassium cations.

The PCR process is a sequence of -20-50 cycles, each of them consisting of the following three steps:
I. Denaturation:
   The reaction mix is heated to a temperature of 94-96°C for 20-30 seconds. In the first cycle, this step can take up to 15 minutes (Initialization). The purpose of these high temperatures is to annihilate the hydrogen bonds between the two strands of the double-stranded DNA.
II. Primer annealing:
   The temperature is lowered for ca. 30 seconds to a temperature which is specific for the annealing of the primers to the single-stranded DNA (∼50-65°C). Too high temperatures lead to excessive thermal movement; hence the primers can't bind to the DNA. Temperatures which are too low forward unspecific binding of the primers to sequences of the DNA which are not entirely complementary.
III. Extension/ Elongation:
   The temperature is increased again to a temperature at which the (Taq) polymerase works best (∼70-80°C). The polymerase uses the dNTPs to synthesize new DNA strands which are complementary to those strands which are tagged by the primers. It starts at the 3'-end of the primer. If everything works fine, the target DNA in the reaction mix is duplicated in each cycle.

The PCR can also be used to quantify the amount of DNA or mRNA fragments in a sample. In this case, a real-time Polymerase Chain Reaction (qPCR) has to be carried out which relies on the basic PCR.

The procedure, described in US 6180349, follows the general principle of polymerase chain reaction; its key feature is that the amplified DNA is detected as the reaction progresses in real time, a new approach compared to standard PCR, where the product of the reaction is detected at its end. Two common methods for detection of products in real-time PCR are: (1) non-specific fluorescent dyes that intercalate with any double-stranded DNA, and (2) sequence-specific DNA probes consisting of oligonucleotides that are labeled with a fluorescent reporter which permits detection only after hybridization of the probe with its complementary DNA target.

In a commonly used variation of the above mentioned second (2) method, also described in US 6180349, the probe is labeled by two fluorophors, a reporter and a quencher, and has to be designed in such a way that it binds to the target DNA strands. This binding takes place during the primer annealing phase. If the probe is activated by a specific wave length during this phase, the fluorescence of the reporter is suppressed due to the spatial vicinity of reporter dye and quencher dye as the reporter releases its energy to the quencher. The underlying concept of this energy transfer is called FRET (fluorescence resonance energy transfer). During the elongation phase the polymerase eliminates the probe which is hydrolysed. Thus the distance between reporter dye and quencher dye increases and the reporter begins to fluoresce. The higher the number of templates, the higher the number of redundant reporter molecules, therefore the intensity of the fluorescence is a measure of the initial number of target molecules.

Plotting the fluorescence intensity after each cycle against the cycle number leads to a so-called sigmoid function which consists of three different parts:
I. Background:
   The number of target molecules and therefore the fluorescence intensity is very small during the first cycles. The fluorescence appears to be constant in the beginning, because the intensity caused by the amplified template is dominated by the so-called background fluorescence. The background fluorescence might be caused by impurities and degenerated reactants in the well or the optical subsystem of the PCR machine.
II. Exponential growth:
   During this phase, the fluorescence increases expponentially.
III. Plateau:
   Due to the consumption of available nucleotides and other limitations the synthesis of product slows down at some time. The intensity doesn't increase exponentially any more during the last cycles, but reaches a saturation phase.

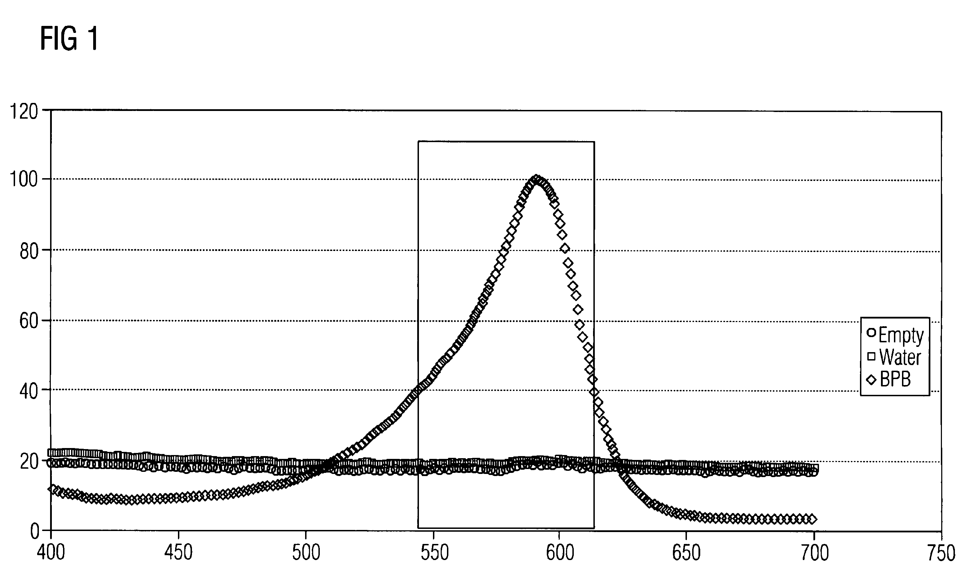

FIG 1 shows the absorption spectrum of Bromphenol blue (BPB). The highlighted wavelength range (from ca. 560 nm to ca. 610 nm) represents the range wherein the absorption of Bromphenol blue is greater than 40% of its maximum absorbance at ca. 580 nm. Thus, Bromphenol blue is suitable as soluble quenching dye for fluorophors with excitation and emission wavelengths from ca. 540 nm to ca. 610 nm. Since Bromphenol blue has a narrow absorption maximum it is particularly well suited for multiplex assay with further detection channels below 560 nm or above 610 nm.

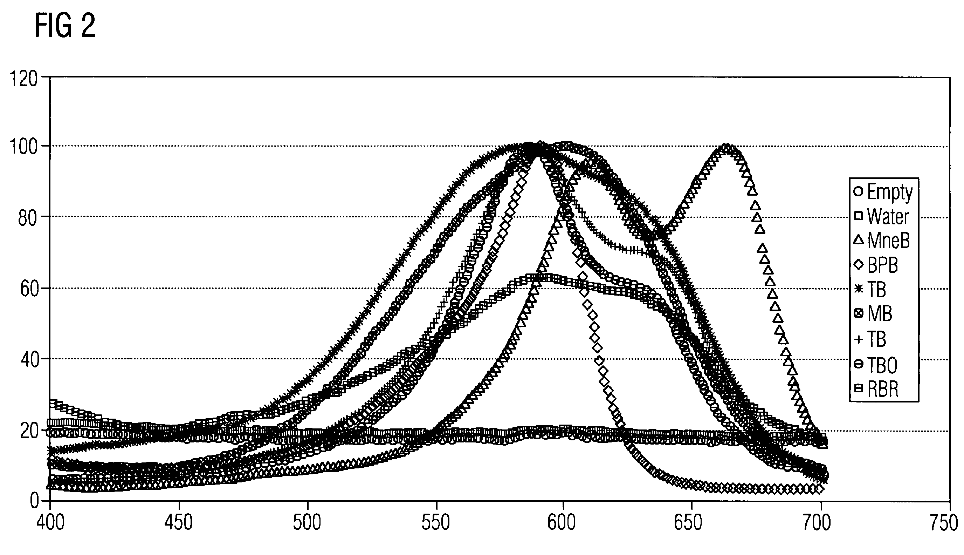

FIG 2 shows absorption spectra of Methylene Blue (MneB), Bromphenol Blu (BPB), Tryptan Blue (TB), Methyl Blue (MB), Toluidine Blue (TBO), New Methylene Blue (TB), Remazol Blue (RBR).

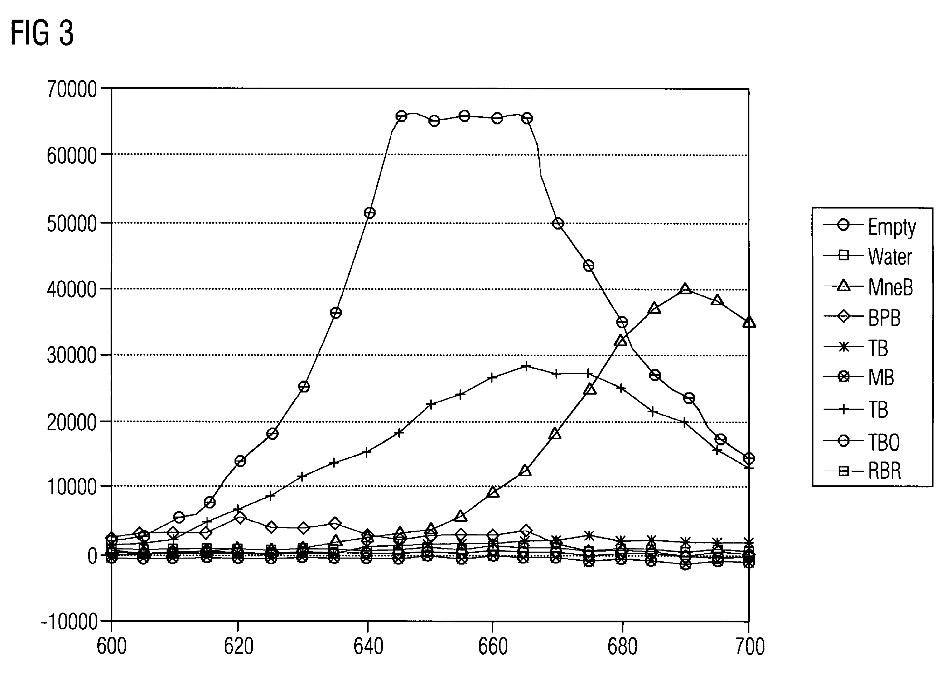

FIG 3 shows fluorescence spectra of Methylene Blue (MneB), Bromphenol Blu (BPB), Tryptan Blue (TB), Methyl Blue (MB), Toluidine Blue (TBO), New Methylene Blue (TB), Remazol Blue (RBR). It can be seen that Bromphenol Blue has a low fluorescence across the tested spectrum.

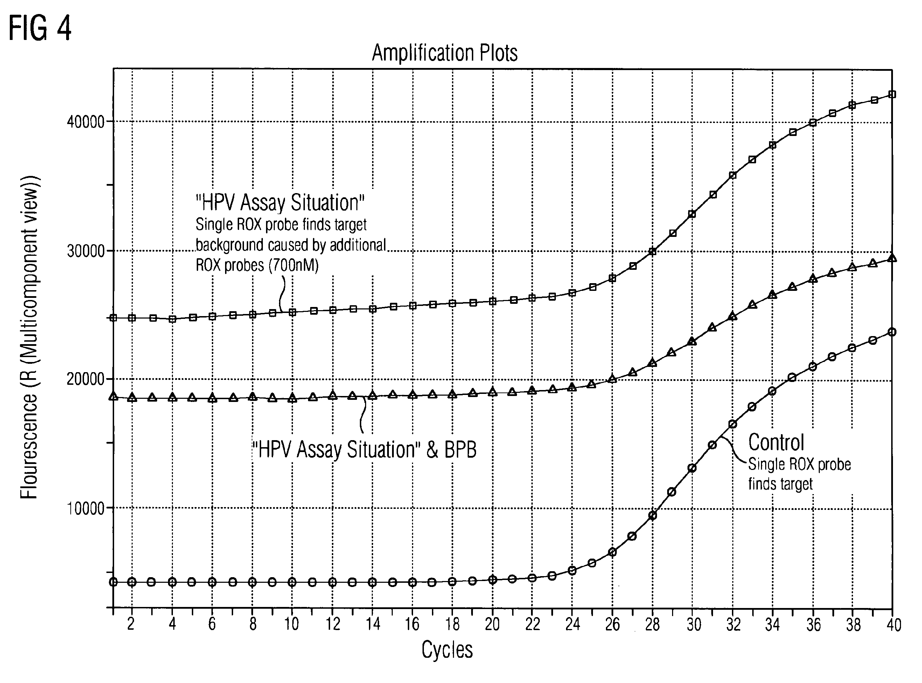

FIG 4 shows reduced background through BPB: Circle symbols show a control of a single human papilloma virus (HPV) specific probe in a HPV assay with low background and strong specific signal with the characteristic sigmoid curve. Square symbols show "HPV Assay Situation", i.e. a single ROX-labeled probe finds target, and background is caused by additional ROX probes (700 nM). Triangle symbols show the situation when Bromphenol Blue (0.04 % w/v, stock diluted 1:200) is added: both background and signal are reduced effectively. This is desirable, because the sensor starts to become oversaturated and is no longer linear at signal intensities of over 30.000 relative units.

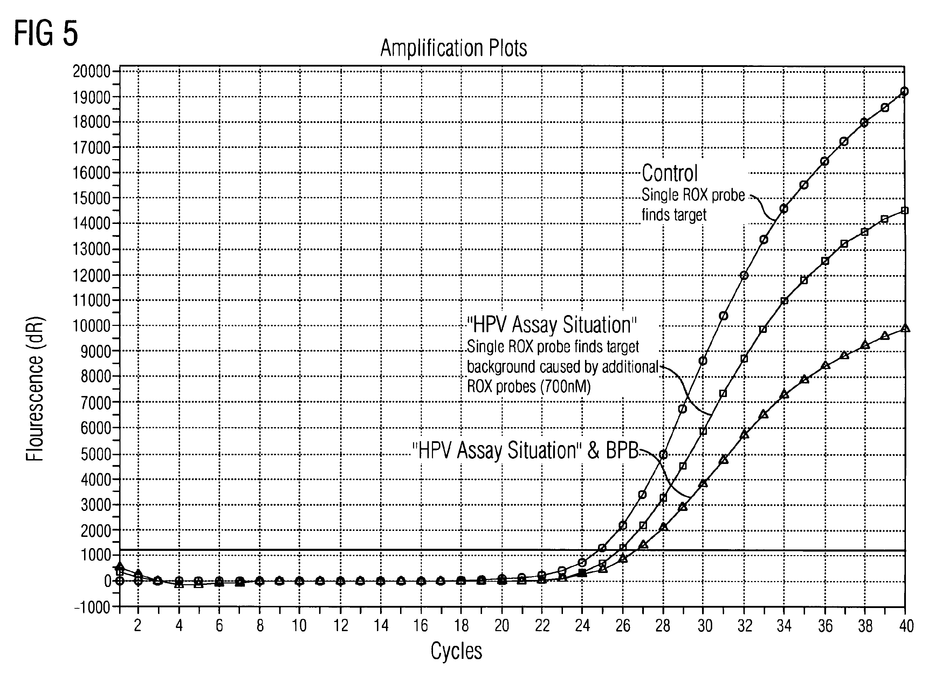

FIG 5 shows data from the same experiment as FIG 4, but with normalized background, thus showing specific signal strengths. The control signal is the strongest specific signal, with a weaker specific signal for the "HPV Assay situation" and the weakest specific signal for the situation when Bromphenol Blue is added. However, the reduced specific signal when Bromphenol Blue is added is still strong enough to accurately determine the concentration of the HPV nucleic acid analyte.

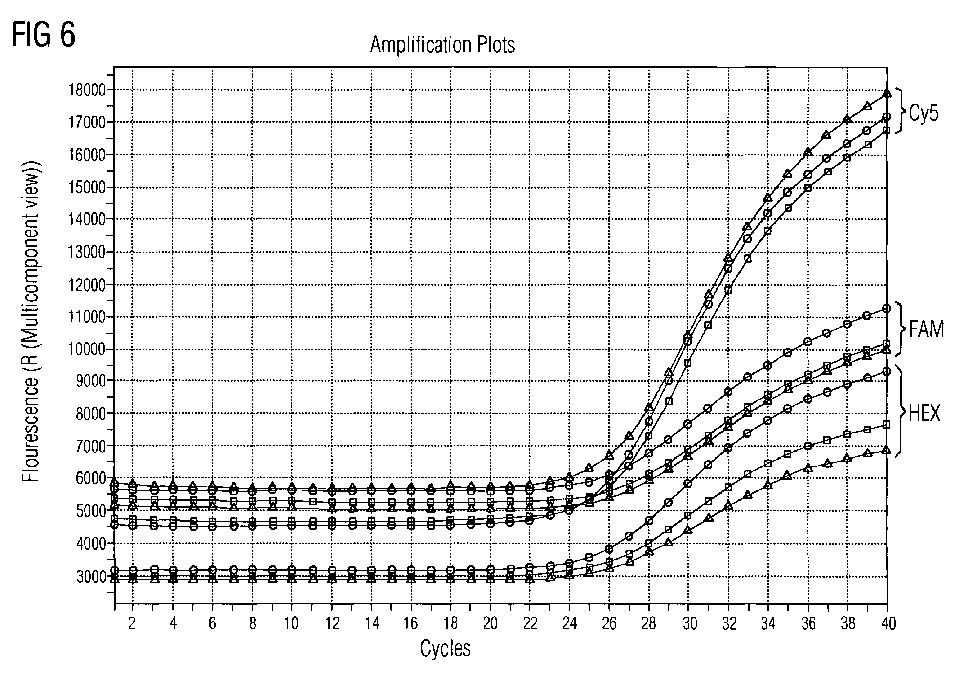

FIG 6 shows the impact of Bromphenol Blue on other Detection Channels: Circle symbols show a control of a single HPV specific probe in a HPV assay in the CY5, FAM and HEX channel respectively. The addition of Bromphenol Blue does not have a significant impact on signals in thses channels, which is desirable in multiplex assays, where other channels should preferably remain unaffected by addition of the soluble quenching dye.

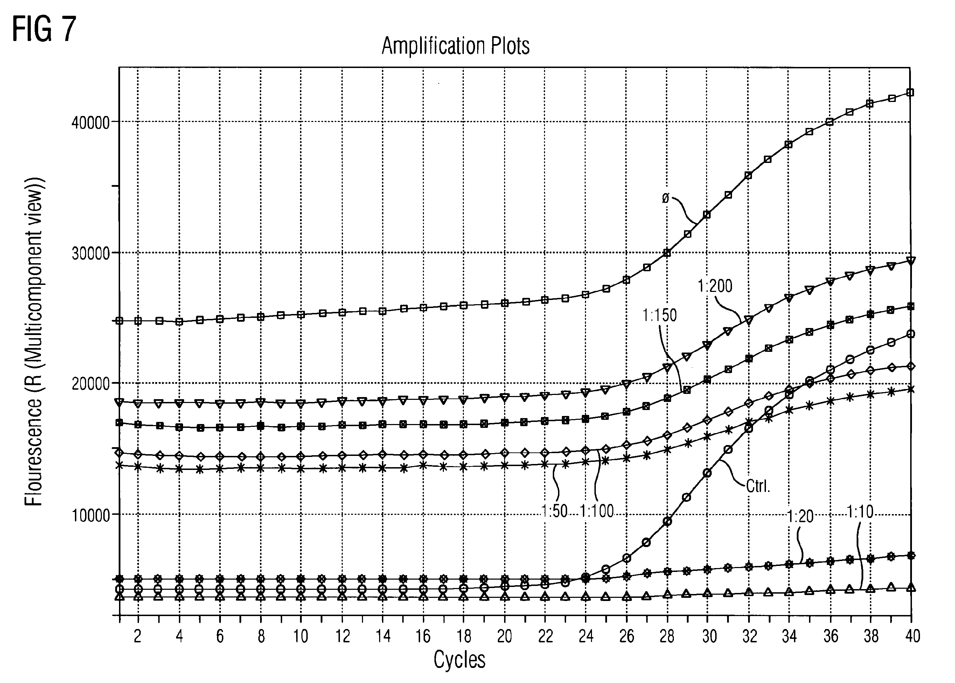

FIG 7 shows a dilution series of the Bromphenol Blue quenching dye (0.04 % w/v, stock diluted from 1:200 to 1:10). A dilution of 1:200 still effectively reduces background. The control shows signals from single HPV specific probe in a HPV assay with low background and strong specific signal. The zero dilution (0 symbol, no BPB added) shows a single ROX-labeled probe finding target, and background is caused by additional ROX probes (700 nM).

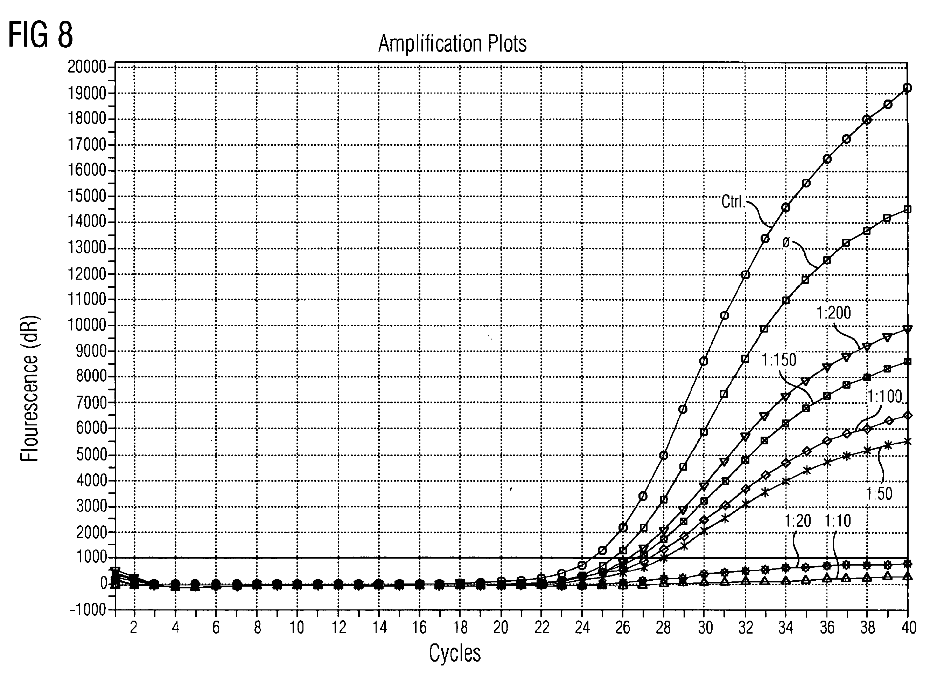

FIG 8 shows data from the same experiment as FIG 7, but with normalized background, thus showing specific signal strengths. The control signal (single probe, no BPB) is the strongest specific signal, with a weaker specific signal for the zero dilution and the increasingly weaker specific signals for increasingly higher BPB concentrations. It can be seen that at dilutions of 1:10 or 1: 20, the specific signal becomes very weak.

In experiments using ROX labeled probes or CAL Fluor Red610 labeled probes, a 0.04%-stock solution of Bromphenol blue (w/v) was used as soluble quenching dye which was diluted 1:10 to 1:200 in experiments (Figs. 7 and 8). It can be assumed that a dilution of 1:1000 can still be effective (corresponding to 0.00004% (w/v). An amount of 0.004% (1:10-dilution) was the upper limit for Bromphenol blue, since the dampening is such that an increase in fluorescence is barely discernible. It is remarked that appropriate dilutions for other soluble quenching dyes may be different, depending on the fluorescent label dye used (i.e. strong or weak fluorphor) and the absolute absorption coefficient of the soluble quenching dye. However, an appropriate dilution may be determined by a simple dilution series which is within the scope of skill of the person skilled in the art.

## Claims

1. Method for determining the presence of a test nucleic acid in a sample, comprising the steps of
a) amplifying the test nucleic acid by an amplification reaction,
b) labeling the amplified nucleic acid by providing a fluorescent dye label with an excitation wavelength and an emission wave length,
c) adding a soluble quenching dye, said soluble quenching dye having an absorption of at least 40% of its maximal absorbance at the excitation wavelength and/or at the emission wave length of the fluorescent dye label,
d) detecting the labeled nucleic acid dye by detecting emitted light at the emission wavelength of the label,
wherein the concentration of said soluble quenching dye is 1 %(w/v) or less, wherein the fluorescent dye label has an excitation wavelength and/or an emission wave length between 340 and 710 nm, and wherein the soluble quenching dye is selected from the group consisting of Bromphenol Blue, Tryptan Blue, Methyl Blue, and Remazol Blue.

2. Method according to claim 1 wherein the amplification reaction is a PCR reaction.

3. Method according to claim 1 or 2, wherein the fluorescent dye label is an intercalating dye.

4. Method according to claim 1 or 2, wherein the fluorescent dye label is bound to a nucleic acid probe capable of specifically binding the test nucleic acid.

5. Method according to any of the preceding claims, wherein the presence at least one further test nucleic is determined, wherein said at least one further test nucleic acid is labeled with at least one further fluorescent dye label and wherein said quenching dye has an absorption of at most 50% of its maximal absorbance or less at the excitation wavelength and/or at the emission wave length of said at least one further fluorescent dye label.

6. Method according to any of the preceding claims, wherein the concentration of said soluble quenching dye is 50% or less of the concentration of said fluorescent dye label.

## Patentansprüche

1. Verfahren zur Bestimmung der Gegenwart einer Test-Nukleinsäure in einer Probe, das folgende Schritte umfasst:
a) Amplifizieren der Test-Nukleinsäure durch eine Amplifizierungsreaktion,
b) Markieren der amplifizierten Nukleinsäure durch Bereitstellen eines Fluoreszenzfarbstoff-Markers mit einer Anregungswellenlänge und einer Emissionswellenlänge,
c) Zugeben eines löslichen Quenchfarbstoffs, wobei der lösliche Quenchfarbstoff eine Absorption von mindestens 40% seiner maximalen Extinktion bei der Anregungswellenlänge und/oder bei der Emissionswellenlänge des Fluoreszenzfarbstoff-Markers aufweist,
d) Detektieren des markierten Nukleinsäure-Farbstoffs durch Detektieren von emittiertem Licht bei der Emissionswellenlänge des Markers,
wobei die Konzentration des löslichen Quenchfarbstoffs 1% (w/v) oder weniger beträgt, wobei der Fluoreszenzfarbstoff-Marker eine Anregungswellenlänge und/oder eine Emissionswellenlänge zwischen 340 und 710 nm aufweist und wobei der lösliche Quenchfarbstoff aus der Gruppe bestehend aus Bromphenolblau, Tryptanblau, Methylblau und Remazolblau ausgewählt wird.

2. Verfahren nach Anspruch 1, bei dem es sich bei der Amplifizierungsreaktion um eine PCR-Reaktion handelt.

3. Verfahren nach Anspruch 1 oder 2, bei dem es sich bei dem Fluoreszenzfarbstoff-Marker um einen interkalierenden Farbstoff handelt.

4. Verfahren nach Anspruch 1 oder 2, bei dem der Fluoreszenzfarbstoff-Marker an eine Nukleinsäuresonde, die spezifisch an die Test-Nukleinsäure binden kann, gebunden ist.

5. Verfahren nach einem der vorhergehenden Ansprüche, bei dem die Gegenwart mindestens einer weiteren Test-Nukleinsäure bestimmt wird, wobei die mindestens eine weitere Test-Nukleinsäure mit mindestens einem weiteren Fluoreszenzfarbstoff-Marker markiert wird und wobei der Quenchfarbstoff eine Absorption von höchstens 50% seiner maximalen Extinktion oder weniger bei der Anregungswellenlänge und/oder bei der Emissionswellenlänge des mindestens einen weiteren Fluoreszenzfarbstoff-Markers aufweist.

6. Verfahren nach einem der vorhergehenden Ansprüche, bei dem die Konzentration des löslichen Quenchfarbstoffs 50% oder weniger der Konzentration des Fluoreszenzfarbstoff-Markers beträgt.

## Revendications

1. Procédé de détermination de la présence d'un acide nucléique de test dans un échantillon, comprenant les stades de
a) amplification de l'acide nucléique de test par une réaction d'amplification,
b) marquage de l'acide nucléique amplifié en fournissant un marqueur colorant fluorescent ayant une longueur d'onde d'excitation et une longueur d'onde d'émission,
c) addition d'un colorant soluble désactivant, le colorant soluble désactivant ayant une absorption d'au moins 40 % de son absorbance maximum à la longueur d'onde d'excitation et/ou à la longueur d'onde d'émission du marqueur colorant fluorescent,
d) détection du colorant d'acide nucléique marqué en détectant de la lumière émise à la longueur d'onde d'émission du marqueur,
dans lequel la concentration du colorant soluble désactivant est inférieure ou égale à 1% (p/v), le marqueur colorant fluorescent ayant une longueur d'onde d'excitation et/ou une longueur d'onde d'émission entre 340 et 710 nm, et le colorant soluble désactivant étant choisi dans le groupe consistant en le bleu de bromophénol, le bleu de tryptane, le bleu de méthyle, et le bleu de rémazol.

2. Procédé suivant la revendication 1 dans lequel la réaction d'amplification est une réaction PCR.

3. Procédé suivant la revendication 1 ou 2, dans lequel le marqueur colorant fluorescent est un colorant d'intercalation.

4. Procédé suivant la revendication 1 ou 2, dans lequel le marqueur colorant fluorescent est fixé à une sonde d'acide nucléique apte à fixer spécifiquement l'acide nucléique de test.

5. Procédé suivant l'une quelconque des revendications précédentes, dans lequel la présence d'au moins un autre nucléique de test est déterminée, le au moins un autre acide nucléique de test étant marqué par au moins un autre marqueur colorant fluorescent et le colorant désactivant ayant une absorption d'au plus 50 % de son absorbance maximum ou moins à la longueur d'onde d'excitation et/ou à la longueur d'onde d'émission du au moins un autre marqueur colorant fluorescent.

6. Procédé suivant l'une quelconque des revendications précédentes, dans lequel la concentration du colorant soluble désactivant est inférieure ou égale à 50 % du marqueur colorant fluorescent.
